# EUROPÄISCHE PATENTSCHRIFT

(11) **EP 1 772 077 B1**
(45) Veröffentlichungstag und Bekanntmachung des Hinweises auf die Patenterteilung: **15.04.2009**
(21) Anmeldenummer: 06018809.1
(22) Anmeldetag: 08.09.2006
(51) Int. Cl.: A47F 1/10

(54) **Vorrichtung zum Bevorraten und definierten Abgeben von Gehörschutzstöpsel**
Storing and dispensing device for earplugs
Dispositif de conditionnement et de distribution du bouchons auriculaires

(30) Priorität: 07.10.2005 DE 102005048057
(43) Veröffentlichungstag der Anmeldung: 11.04.2007
(73) Patentinhaber: Uvex Arbeitsschutz GmbH, 90766 Fürth (DE)
(72) Erfinder: Brück, Stefan, 90419 Nürnberg (DE)
(74) Vertreter: Schneck, Herbert

(56) Entgegenhaltungen:
- EP-A2- 1 074 236
- WO-A-94/09456
- WO-A2-02/00155
- FR-A- 1 101 989
- US-A- 5 322 185

## Beschreibung

Die Erfindung richtet sich auf eine Vorrichtung zur Bevorratung und dosierten Abgabe von Gehörschutzstöpseln umfassend einen Vorratsbehälter, eine den Boden des Vorratsbehälters bildende Drehscheibe mit einer Mehrzahl von auf einer Kreisbahn angeordneten durchgehenden Ausnehmungen, einen die Unterseite der Ausnehmung abdeckenden horizontalen Zwischenboden mit einer im Bereich der Kreisbahn der Ausnehmungen liegenden Durchlassöffnung und ein manuell betätigbares Getriebe als Drehantrieb für den Drehteller.

Eine derartige Vorrichtung ist bekannt aus US 5,285,925 B1. Bei dieser bekannten Vorrichtung ist eine seitlich vorstehende Drehkurbel vorgesehen, welche über ein Umlenkgetriebe den Drehteller antreibt. Diese Ausgestaltung ist ästhetisch nicht befriedigend und weist darüber hinaus den gravierenden Nachteil auf, dass die vorstehende Kurbel beim unachtsamen Vorbeigehen zu Verletzungen fuhren kann. Das verwendete Getriebe ebenso wie die anderen Komponenten der vorbekannten Vorrichtung sind vergleichsweise kostenaufwändig in der Herstellung und zeitaufwändig in der Montage.

Andere bekannte Vorrichtungen wie zum Beispiel in EP 107 42 36 A oder WO 02/00 155 A offenbart sind weisen einen zentralen Drehantrieb für den Drehteller auf, sodass eine Untersetzung nicht realisierbar ist und darüber hinaus der Benutzer einen relativ weit innenliegenden Drehknopf betätigen muss, was die Handhabung nicht fördert.

Hiervon ausgehend liegt der Erfindung die Aufgabe zugrunde, eine Vorrichtung der eingangs genannten Art so auszugestalten, dass sie kostengünstig herstellbar und montierbar ist, wartungsfrei ist und sich leicht betätigen lässt, wobei eine definierte und kontrollierte Abgabe der einzelnen Gehörschutzstöpsel gewährleistet und ein ansprechendes Design realisierbar sein soll.

Diese Aufgabe wird erfindungsgemäß dadurch gelöst, dass ein Drehknopf an der Unterseite des Gehäuses um eine vertikale Drehachse drehbar und zur Drehachse des Drehtellers nach vorne versetzt angeordnet ist.

Durch diese Ausgestaltung wird ein sehr ansprechendes Design erreicht. Die Gefahr von Verletzungen durch seitlich herausstehende Teile wird vermieden und aufgrund des relativ weit vorne, das heißt nahe am Benutzer liegenden Drehknopfes ist eine leichte Bedienung möglich.

In einer weiteren Ausgestaltung der Erfindung ist vorgesehen, dass an der Oberseite des Drehknopfes und an der Unterseite des Drehtellers jeweils ein Zahnkranz angeordnet ist, wobei die Zahnkränze unter Ausbildung eines Getriebes ineinander greifen.

Hierdurch wird einerseits eine reine Übertragung der Drehbewegung bewerkstelligt, die eine Verlagerung des Drehknopfes nach vorne ermöglicht, andererseits ist es möglich eine Unter- oder Übersetzung zu realisieren.

Der Zahnkranz des Drehtellers kann an einem Übertragungselement angeordnet sein, welches mit dem Drehteller drehfest, aber axial lösbar verbindbar ist, das heißt es wird auf diese Weise eine leichte Montage erzielt, da die Teile einfach ineinander gesteckt werden können.

Vorteilhafter Weise sind in einem Zwischenboden Lagerausnehmungen als Gleitlager für den Drehknopf und das Übertragungselement bzw. den Drehteller derart ausgebildet, dass alle bewegten Komponenten von oben axial einführbar und relativ zueinander positioniert und gelagert sind.

Es müssen dementsprechend keine aufwändigen Lager realisiert werden und die Montage ist extrem einfach und schnell.

Eine kostengünstige Herstellbarkeit wird weiterhin dadurch gefördert, dass der Drehknopf mit seinem Zahnkranz, das Übertragungselement mit seinem Zahnkranz und die Drehscheibe jeweils einstückig aus Kunststoff gespritzt werden können.

Günstiger Weise ist an der Unterseite des mittleren Gehäuseabschnitts ein nach vorne offenes Auffanggehäuse angeordnet und auf den mittleren Gehäuseabschnitt ein transparenter Vorratsbehälter aufgesetzt. Hierdurch kann überprüft werden, ob noch ein ausreichender Vorrat von Gehörschutzstöpseln vorhanden oder ein Nachfüllen erforderlich ist.

Am Drehteller sind Stegpaare derart angeordnet, dass durch den in Drehrichtung jeweils vorne liegenden Steg eine Blattfeder ausgelenkt wird, welche bei weiterer Drehbewegung gegen den in Drehrichtung hinten liegenden Steg schlägt. Hierdurch wird ein Geräusch erzeugt, welches dem Benutzer signalisiert, dass er die Drehbewegung beenden kann und ein Gehöhrschutzstöpsel ausgeworfen wird.

Nachfolgend wird die Erfindung anhand eines bevorzugten Ausführungsbeispiels in Verbindung mit der Zeichnung näher erläutert. Dabei zeigen:

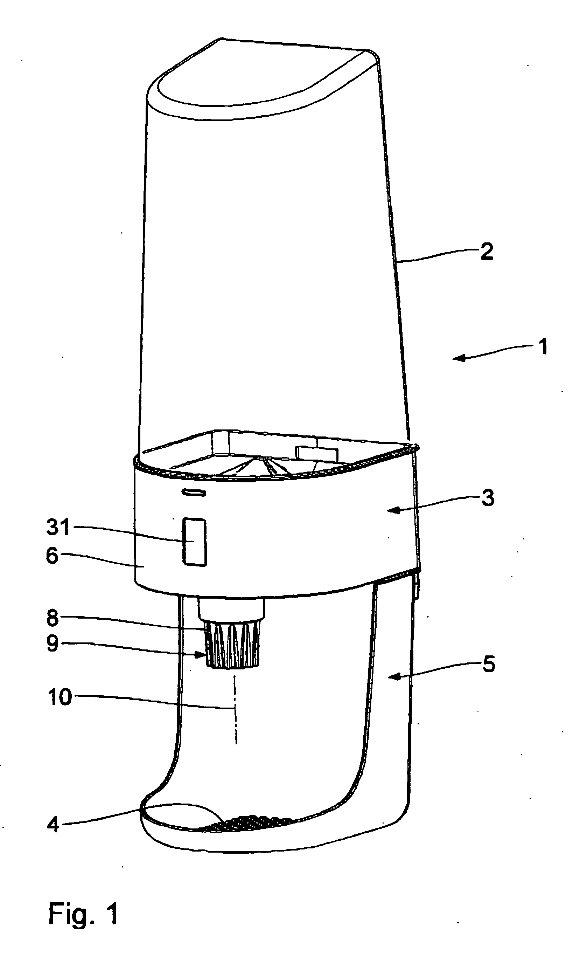

- Fig. 1: eine perspektivische Ansicht einer erfindungsgemäßen Vorrichtung,
- Fig. 2: eine Explosionsdarstellung,

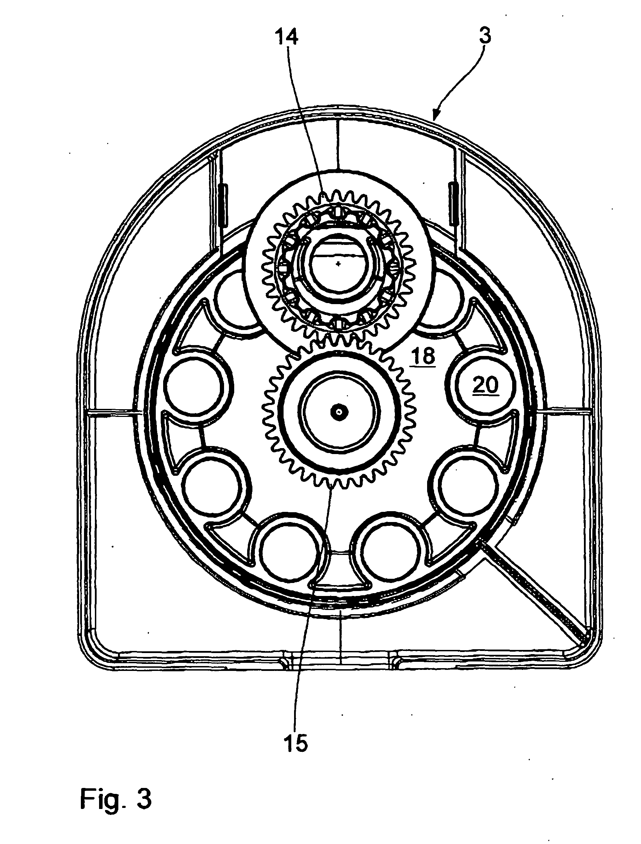

- Fig. 3: eine Ansicht des Drehtellers und dessen Antrieb,
- Fig. 4: eine Seitenansicht zur Veranschaulichung des Kabelvorgangs und
- Fig. 5: eine weitere Seitenansicht von schräg oben.

Eine erfindungsgemäße Vorrichtung 1 zur Bevorratung und definierten Abgabe von Gehörschutzstöpseln umfasst einen transparenten Vorratsbehälter 2, einen mittleren Gehäuseabschnitt 3 und ein nach vorne offenes, mit einem vertikalen Boden 4 versehenes Auffanggehäuse 5.

Im vorderen Bereich 6 des mittleren Gehäuseabschnitts 3 erstreckt sich von dessen Boden 7 weg ein mit Griffrippen 8 versehener Drehknopf 9, welcher um eine Achse 10 schwenkbar ist.

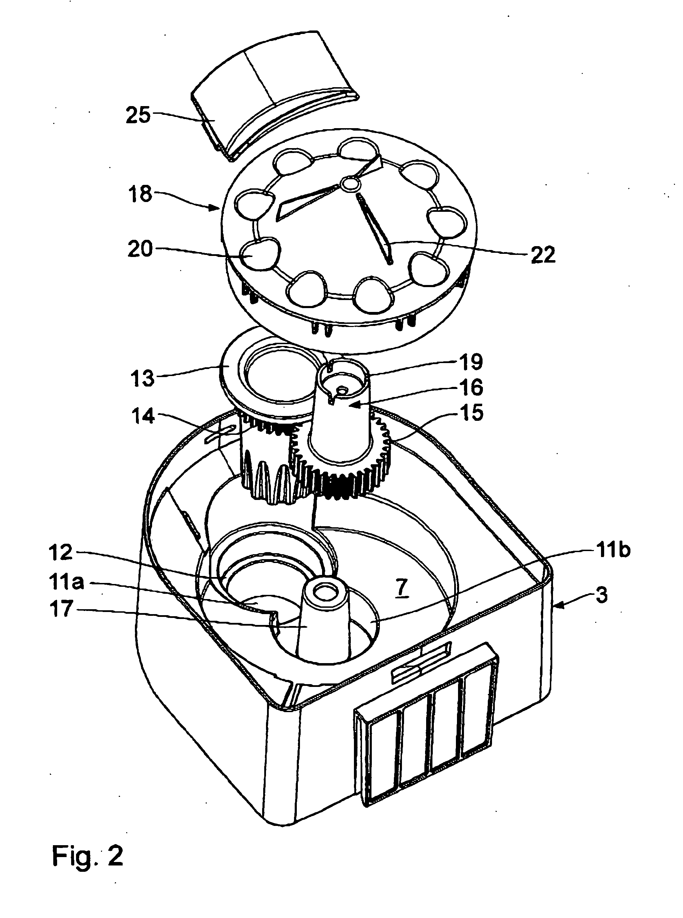

In Fig. 2 ist der innere Aufbau des mittleren Gehäuseabschnitts 3 im Einzelnen dargestellt. Hieraus ist erkennbar, dass der Drehknopf 9 in einer Ausnehmung 11a auf einer diese umgebenden Ringschulter 12 bzw. über einen Ringkragen 13, der im Bereich des Bodens 7 aufliegt, gelagert ist. Unterhalb der Ringkragen 13 ist ein Zahnkranz 14 ausgebildet, der mit einem weiterem Zahnkranz 15 eines Übertragungselements 16 kämmt, welches auf einem Konus 17 drehbar gelagert ist, der seinerseits in einer Ausnehmung 11b angeordnet ist.

Das Übertragungselement 16 dient zur Übertragung der Drehbewegung des Drehknopfs 9 auf einen Drehteller 18 der über in der Zeichnung nicht dargestellte Rippenvorsprünge und Ausnehmungen 19 des Übertragungselements 16 mit diesem drehfest, aber axial lösbar verbunden ist.

Aus dem Vorstehenden wird deutlich, dass die beschriebenen Teile an dem mittleren Gehäuseabschnitt 3 durch einfaches axiales Einsetzen von oben montierbar sind.

Der Drehteller 18 weist eine Mehrzahl von durchgehenden Ausnehmungen 20 auf. Er ist nach oben konisch verlaufend ausgebildet, sodass in dem Vorratsbehälter 2 befindliche Gehörschutzstöpsel 21 in die Ausnehmungen, unterstützt durch die Leitstege 22, die sich von der Mitte des Drehtellers 18 radial nach außen erstrecken, gelenkt werden.

Die Unterseite der Ausnehmungen 20 ist durch den Zwischenboden 7 verschlossen, sodass die Gehörschutzstöpsel zunächst in den Ausnehmungen 20 verbleiben und bei einer Drehbewegung des Drehtellers 18 in den Ausnehmungen 20 im Kreis bewegt werden.

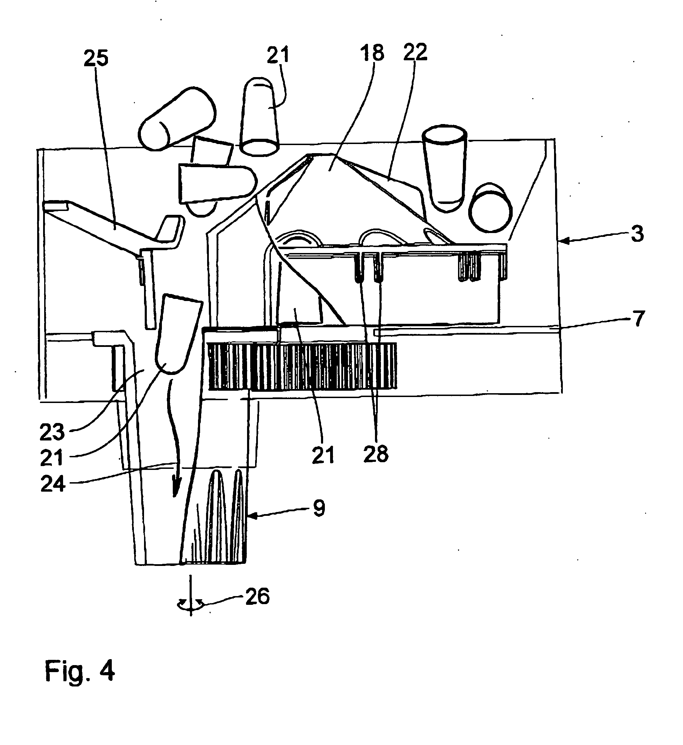

In der so definierten Kreisbahn weist der Boden 7, wie insbesondere aus Fig. 4 erkennbar, eine Durchlassöffnung 23 auf. Wenn eine Ausnehmung 20 diese Durchlassöffnung 23 erreicht, fällt ein Gehörschutzstöpsel 21 in Richtung des Pfeils 24 nach unten heraus auf den Boden 4 und kann von dort bequem entnommen werden.

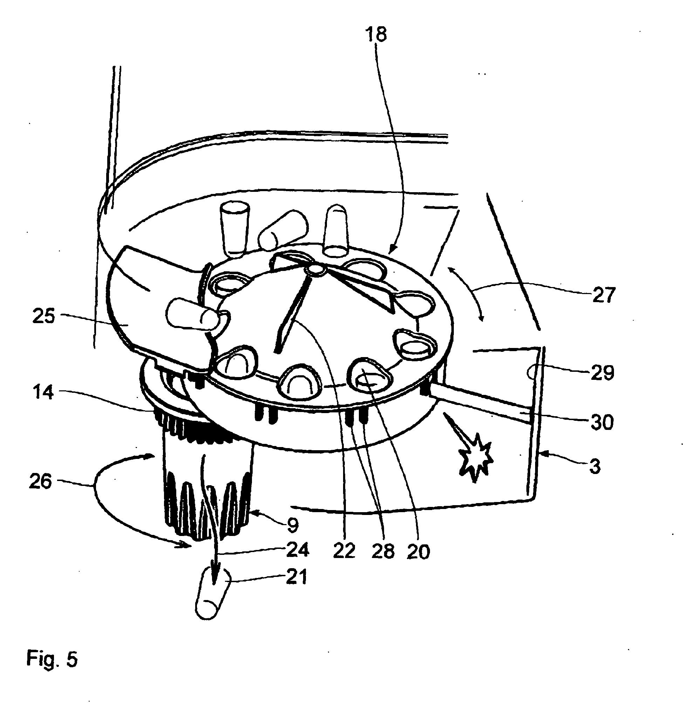

Eine Abdeckung 25 oberhalb der Durchlassöffnung 23 verhindert ein unkontrolliertes direktes Durchfallen des Vorrats von Gehörschutzstöpseln 21. Eine Abgabe ist dementsprechend nur so möglich, dass die Gehörschutzstöpsel 21 zunächst in eine Ausnehmung 20 fallen und einer Drehbewegung in Richtung der Pfeile 26 bzw. 27 in Fig. 5 unterworfen werden.

An der Unterseite des Drehtellers 18 sind im Abstand voneinander jeweils Paare von Stegen 28 angeordnet, wobei sich radial von der Gehäuseinnenwand 29 nach innen eine Blattfeder 30 in die Bewegungsbahn der Stege 28 erstreckt. Wenn der Drehteller 18 in Richtung des Doppelpfeils 27 gedreht wird, lenkt der in Drehrichtung jeweils vorne liegende Steg 28 jedes Steg-Paares das freie Ende der Blattfeder 30 aus, sodass diese bei einer Weiterbewegung freigegeben wird und zurückfedert und dabei gegen den in Drehrichtung folgenden zweiten Steg schlägt und so ein Geräusch erzeugt, welches dem Benutzer signalisiert, dass ein Ohrstöpsel 21 durch die Durchlassöffnung 23 gefallen ist, weil die Stege 28 so angeordnet sind, dass das Geräusch jeweils dann erzeugt wird, wenn eine Ausnehmung 20 die Durchlassöffnung 23 passiert bzw. passiert hat.

Zur Kontrolle des Abgabevorgangs kann an der Vorderseite des Zwischengehäuses 3 ein Sichtfenster 31 vorgesehen sein.

## Patentansprüche

1. Vorrichtung (1) zur Bevorratung und dosierten Abgabe von Gehörschutzstöpseln (21) umfassend
- einen Vorratsbehälter (2),
- einen den Boden des Vorratsbehälters (2) bildenden Drehteller (18) mit einer Mehrzahl von auf einer Kreisbahn angeordneten durchgehenden Ausnehmungen (20),
- einen die Unterseite der Ausnehmung abdeckenden horizontalen Zwischenboden (7) mit einer im Bereich der Kreisbahn der Ausnehmung (20) liegenden Durchlassöffnung (23) und
- ein manuell betätigbares Getriebe (14, 15) als Drehantrieb für den Drehteller (18),
**dadurch gekennzeichnet, dass** ein Drehknopf (9) an der Unterseite des Gehäuses (2, 3) um eine vertikale Drehachse (D1) drehbar und zur Drehachse (D2) des Drehtellers (18) nach vorne versetzt angeordnet ist.

2. Vorrichtung nach Anspruch 1, **dadurch gekennzeichnet, dass** an der Oberseite des Drehknopfes (9) und an der Unterseite des Drehtellers (18) jeweils ein Zahnkranz (14 bzw. 15) angeordnet ist, wobei die Zahnkränze 14 bzw. 15) unter Ausbildung eines Getriebes ineinander greifen.

3. Vorrichtung nach Anspruch 2, **dadurch gekennzeichet, dass** der Zahnkranz (15) des Drehtellers (18) an einem Übertragungselement (16) angeordnet ist, welches mit dem Drehteller (18) drehfest, aber axial lösbar verbindbar ist.

4. Vorrichtung nach Anspruch 1, **dadurch gekennzeichnet, dass** in einem Zwischenboden (7) Lagerausnehmungen (11a, 11b) als Gleitlager für den Drehknopf (9) und das Übertragungselement (16) bzw. den Drehteller (18) derart ausgebildet sind, dass alle bewegten Komponenten von oben axial einführbar und relativ zueinander positioniert und gelagert sind.

5. Vorrichtung nach Anspruch 1, **dadurch gekennzeichnet, dass** der Drehknopf (9) mit seinem Zahnkranz (14), das Übertragungselement (16) mit seinem Zahnkranz (15) und der Drehteller (18) jeweils einstückig aus Kunststoff gespritzt sind.

6. Vorrichtung nach Anspruch 1, **dadurch gekenntzeichnet, dass** an der Unterseite des mittleren Gehäuseabschnitts (3) ein nach vorne offenes Auffanggehäuse (5) angeordnet ist.

7. Vorrichtung nach Anspruch 1, **dadurch gekennzeichnet, dass** auf den mittleren Gehäuseabschnitt (3) ein transparenter Vorratsbehälter (2) aufgesetzt ist.

8. Vorrichtung nach Anspruch 1, **dadurch gekennzeichnet, dass** am Drehteller Stegpaare derart angeordnet sind, dass durch den in Drehrichtung jeweils vorne liegenden Steg (28) eine Blattfeder (30) ausgelenkt wird, welche bei weiterer Drehbewegung gegen den in Drehrichtung hinten liegenden Steg (28) schlägt.

## Claims

1. Device (1) for storing and dispensing doses of ear plugs (21), the device (1) comprising
- a storage container (2);
- a turntable (18) which forms the bottom of the storage container (2) and comprises a plurality of continuous cutouts (20) arranged along a circular path;
- a horizontal intermediate bottom (7) which covers the underside of the cutout and comprises a through opening (23) disposed in the region of the circular path of the recess (20); and
- a manually operable gear (14, 15) as a rotary drive for the turntable (18);
**characterized in that** a rotary knob (9) is arranged on the underside of the housing (2, 3) for rotation about a vertical axis of rotation (D1) and is forwardly offset relative to the axis of rotation (D2) of the turntable (18).

2. Device according to claim 1, **characterized in that** on the upper side of the rotary knob (19) and on the underside of the turntable (18), there is arranged in each case one gear ring (14 or 15, respectively), wherein the gear rings (14 or 15, respectively) engage with one another so as to form a gear.

3. Device according to claim 2, **characterized in that** the gear ring (15) of the turntable (18) is disposed on a transmission element (16) which is non-rotatably but axially releasably connectable to the turntable (18).

4. Device according to claim 1, **characterized in that** bearing recesses (11a, 11b) are formed in an intermediate bottom (7) as slide bearings for the rotary knob (9) and the transmission element (16) or the turntable (18), respectively, in such a way that all moved components are axially insertable from above and are positioned and mounted relative to each other.

5. Device according to claim 1, **characterized in that** the rotary knob (9) with its gear ring (14), the transmission element (16) with its gear ring (15) and the turntable (18) are in each case injection moulded in one piece from plastics.

6. Device according to claim 1, **characterized in that** on the underside of the central housing portion (3) is arranged a collector housing (5) which is open to the front.

7. Device according to claim 1, **characterized in that** a transparent storage container (2) is placed on the central housing portion (3).

8. Device according to claim 1, **characterized in that** pairs of webs are arranged on the turntable in such a way that a leaf spring (30) is deflected by the web (28) which is in an anterior position when seen in the direction of rotation, with the leaf spring (30), upon further rotation, abutting against the web (28) which is in a posterior position when seen in the direction of rotation.

## Revendications

1. Dispositif (1) de conditionnement et de distribution dosée de bouchons auriculaires (21) comprenant
- un réservoir (2),
- un plateau tournant (18) formant le fond du réservoir (2) et doté d'une pluralité d'évidements (20) traversants disposés sur une trajectoire circulaire,
- un fond intermédiaire (7) horizontal couvrant la face inférieure de l'évidement et doté d'une ouverture de passage (23) située dans la zone de la trajectoire circulaire de l'évidement (20) et
- un engrenage (14, 15) à actionnement manuel sous forme d'entraînement rotatif pour le plateau tournant (18),
**caractérisé en ce qu'**un bouton rotatif (9) au niveau de la face inférieure du bâti (2, 3) est disposé de manière rotative autour d'un axe de rotation vertical (D1) et de manière décalée vers l'avant par rapport à l'axe de rotation (D2) du plateau tournant (18).

2. Dispositif selon la revendication 1, **caractérisé en ce que** respectivement une couronne dentée (14 respectivement 15) est disposée au niveau de la face supérieure du bouton rotatif (9) et au niveau de la face inférieure du plateau tournant (18), les couronnes dentées (14 respectivement 15) prenant l'une dans l'autre en formant un engrenage.

3. Dispositif selon la revendication 2, **caractérisé en ce que** la couronne dentée (15) du plateau tournant (18) est agencée au niveau d'un élément de transmission (16), qui peut être relié au plateau tournant (18) de manière solidaire en rotation, mais axialement amovible.

4. Dispositif selon la revendication 1, **caractérisé en ce que** dans un fond intermédiaire (7), des évidements de logement (11a, 11b) sont réalisés sous forme de palier lisse pour le bouton rotatif (9) et l'élément de transmission (16) ou encore le plateau tournant (18) de telle sorte que tous les composants mobiles peuvent être introduits par le haut et sont positionnés et logés de manière relative l'un par rapport à l'autre.

5. Dispositif selon la revendication 1, **caractérisé en ce que** le bouton rotatif (9) avec sa couronne dentée (14), l'élément de transmission (16) avec sa couronne dentée (15) et le plateau tournant (18) sont chacun moulés par injection d'un seul tenant en plastique.

6. Dispositif selon la revendication 1, **caractérisé en ce qu'**un bâti de réception (5) ouvert vers l'avant est agencé au niveau de la face inférieure de la section médiane du bâti (3).

7. Dispositif selon la revendication 1, **caractérisé en ce qu'**un réservoir transparent (2) est rapporté sur la section médiane du bâti (3).

8. Dispositif selon la revendication 1, **caractérisé en ce que** des paires de nervure sont disposées au niveau du plateau tournant de telle sorte qu'un ressort à lames (30) soit articulé par la nervure (28) située respectivement à l'avant dans le sens de rotation, lequel ressort à lames heurte la nervure (28) située à l'arrière dans le sens de rotation en cas de poursuite du mouvement de rotation.
